# DEMANDE DE BREVET EUROPEEN

(11) **EP 3 549 577 A1**
(43) Date de publication de la demande: **09.10.2019**
(21) Numéro de dépôt: 19167241.9
(22) Date de dépôt: 04.04.2019
(51) Int. Cl.: A61K 9/14, A61K 9/00, A61B 5/00, A61B 5/05, B22F 9/04, H01F 1/00

(54) **PROCEDE DE FABRICATION D'UN FLUIDE BIOCOMPATIBLE COMPORTANT UNE POUDRE DE PARTICULES MAGNETIQUES, FLUIDE BIOCOMPATIBLE COMPORTANT UNE POUDRE DE PARTICULES MAGNETIQUES**

(30) Priorité: 05.04.2018 FR 1852971
(71) Demandeur: Commissariat à l'Energie Atomique et aux Energies Alternatives, 75015 Paris (FR); CENTRE NATIONAL DE LA RECHERCHE SCIENTIFIQUE - CNRS, 75794 Paris Cedex 16 (FR)
(72) Inventeur: MOREL, Robert, 38054 GRENOBLE (FR); DIENY, Bernard, 38054 GRENOBLE (FR); JOISTEN, Hélène, 38054 GRENOBLE (FR)
(74) Mandataire: Cabinet Camus Lebkiri

(57) **Abrégé**

Procédé de fabrication d'un fluide biocompatible comportant une poudre de particules magnétiques de forme allongée possédant une anisotropie magnétique de forme et ayant une granulométrie finale, ladite granulométrie finale étant définie par une première taille moyenne des particules dans une première direction et une deuxième taille moyenne dans une deuxième direction différente de la première direction, ladite granulométrie finale étant en outre définie par une première largeur de distribution des premières tailles et une deuxième largeur de distribution des deuxièmes tailles, ledit procédé comprenant les étapes suivantes :
- A partir d'une poudre de particules magnétiques ayant une granulométrie initiale différente de la granulométrie finale, modification (G1) de la granulométrie initiale par broyage et/ou par frittage de la poudre jusqu'à obtention de la granulométrie finale ;
- Introduction (FL) de la poudre de particules magnétiques dans un fluide biocompatible.

## Description

### DOMAINE TECHNIQUE

L'invention appartient au domaine des fluides biocompatibles comprenant une poudre de particules magnétiques pour l'application d'une vibration magnéto-mécanique basse fréquence. Un premier objet de l'invention est un procédé de fabrication comprenant une poudre de particules magnétiques ayant une granulométrie donnée. Un deuxième objet de l'invention est un fluide biocompatible comprenant une poudre de particules magnétiques pour l'application d'un couple magnéto-mécanique basse fréquence. Le fluide biocompatible selon l'invention peut être utilisé pour la destruction de cellules cancéreuses par la mise en vibration basse fréquence de particules à proximité des cellules cibles.

### ETAT DE L'ART

Dans le domaine des biotechnologies, des particules magnétiques superparamagnétiques de toute petite taille (au plus quelques dizaines de nanomètres) sont de plus en plus utilisées dans diverses applications telles que la délivrance ciblée de molécules de traitement (drug delivery), la séparation de molécules ou de cellules en suspension, les traitements de cancer par hyperthermie, l'ingénierie de tissus cellulaires ou comme agents de contraste, voir par exemple le document "Applications of magnetic nanoparticles in biomedicine", publié dans J. Phys. D: Appl. Phys. 36, R167, 2003 de Q. A. Pankhurst et al.

Les particules le plus souvent utilisées sont des petites particules superparamagnétiques recouvertes d'une enveloppe, inorganique comme par exemple de silice, ou polymère comme par exemple le dextrane ou le polyéthylène glycol (PEG), assurant leur protection et leur biocompatibilité. Les particules peuvent être fonctionnalisées par recouvrement d'une couche de molécules, par exemple pour se fixer de façon ciblée sur une cellule ou un tissu, pour transporter un composé actif ou un médicament, ou pour assurer leur dispersion ou leur mobilité. Suivant les applications visées, ces molécules peuvent assurer leur dispersion ou leur mobilité. Suivant les applications visées, ces particules peuvent avoir des dimensions de quelques nanomètres (agent de contraste IRM), ou de plusieurs micromètres lorsqu'elles sont composées d'un assemblage de particules magnétiques, noyés dans une matrice.

Des approches thérapeutiques nouvelles, notamment pour le traitement du cancer, reposent sur l'exercice d'actions mécaniques sur des espèces biologiques grâce à des particules magnétiques qui peuvent être internalisées, au contact, ou à proximité des milieux à traiter (voir par exemple le document « Biofunctionalized magnetic-vortex microdiscs for targeted cancer-cell destruction » publié dans Nature Materials 9, 165 (2010), de D. Kim et al). La force mécanique exercée par une particule magnétique est la conséquence du couple magnétique qui s'exerce sur elle, qui pour sa part résulte de l'application d'un champ variable en direction ou en module (voir le document « Actuating Soft Matter with Magnetic Torque », publié dans Adv. Funct. Mater. 26, 3859 (2016), de R. M. Erb et al).

Il est connu d'utiliser la vibration magnéto-mécanique basse fréquence de particules magnétiques pour des applications thérapeutiques. Les particules utilisées pour ces applications possèdent une taille proche du micromètre et ne comprennent pas d'oxydes métalliques. Les champs magnétiques utilisés pour la mise en vibration des particules oscillent à une fréquence qui est au plus de quelques dizaines de Hz.

Des particules magnétiques d'oxydes métalliques de petite taille - typiquement quelques dizaines de nm - sont utilisées pour d'autres applications telles que l'hyperthermie. Dans ces applications, des champs magnétiques de plusieurs centaines de kHz sont utilisés pour réchauffement des particules.

Les particules magnétiques requises pour les applications où il s'agit d'induire la vibration magnéto-mécanique pour la destruction de cellules doivent présenter un certain nombre de caractéristiques essentielles :
- Elles doivent présenter une faible aimantation à champ nul, pour éviter l'agglomération ;
- Elles doivent pouvoir être actionnées par un champ de faible amplitude ;
- Pour les particules où l'anisotropie de forme domine, elles doivent être de forme anisotrope pour que le couple magnétique induise une rotation de la particule (et non pas uniquement une rotation de son aimantation) ;
- Elles doivent avoir une taille de l'ordre du micromètre pour que la rotation de la particule induise un effet mécanique sensible sur la cellule ou le milieu visé ;
- Elles doivent être biocompatibles.

Plusieurs documents de l'état de la technique décrivent des particules magnétiques pour l'application d'une vibration basse fréquence, mais les particules connues ne comportent pas l'ensemble des caractéristiques idéalement requises.

La demande de brevet WO 2005011810, « Magnetic particles for therapeutic treatment », déposée par Oxford Instruments Superconductivity Limited, présente une méthode de destruction de cellules par rupture mécanique, où des particules localisées sont soumises à des champs magnétiques de faible intensité et dont l'orientation varie à faible fréquence. Les particules décrites par ce document sont préférentiellement des particules de faible anisotropie magnétique, dont la plus grande dimension est inférieure à 0.5 µm. La forme de ces particules est de préférence ellipsoïdale prolate, ou ellipsoïdale oblate. L'aimantation des particules est stabilisée par l'anisotropie magnétocristalline intrinsèque et/ou par l'anisotropie de forme. Les paramètres de taille et de forme sont choisis pour minimiser le champ magnétique nécessaire à l'application d'une force de 100 pN, jugée nécessaire pour engendrer un effet notable sur les cellules. Aucune méthode de fabrication de ces particules n'est divulguée par ce document.

La demande de brevet WO 2006134365, « Method of providing magnetised particles at a location» déposée par Oxford Instruments Molecular Biotools, décrit des particules magnétiques à structure en vortex, optimisées pour l'application d'une force. Les particules sont composées de Supermalloy (Ni78Fe18Mo4), de permalloy (Ni80Fe20) ou de nickel. Les particules décrites par ce document peuvent posséder des formes différentes :
- Particules sphériques de diamètre compris entre 50 nm et 200 nm ;
- Particules quasi-sphériques ;
- Particules ayant une grande dimension et une petite dimension, la grande dimension étant comprise entre 50 nm et 200 nm, la petite dimension étant supérieure à 5 nm et inférieure à la grande dimension.

Grâce à la structure en vortex, ces particules ont une aimantation à champ nul très faible, ce qui favorise une bonne dispersion, en limitant l'agrégation due à l'interaction magnétique. De plus, ces particules présentent une susceptibilité magnétique élevée, ce qui réduit le champ magnétique nécessaire à leur mise en mouvement. Toutefois ces particules ne sont pas biocompatibles car elles contiennent toutes du nickel.

D'autres documents de l'état de la technique considèrent l'utilisation de particules d'oxyde de fer pour détruire les cellules par une action mécanique, mais dans des régimes de fréquence ou d'intensité de champ magnétique élevés ou encore par l'utilisation de forts gradients de champs magnétiques.

La demande de brevet WO 200137721 décrit une méthode pour induire la mort cellulaire sous l'effet de particules de moins de 100 nm. Les particules sont en contact avec la cellule ou internalisées par endocytose, suivant l'application d'un champ magnétique statique. Le mécanisme d'action des particules n'est pas explicité, mais l'utilisation d'un champ magnétique statique exclut la mise en vibration des particules.

La demande de brevet WO 2014038829 « Procédé d'activation de manière sélective de nanoparticule magnétique et nanoparticule magnétique activé de manière sélective » déposée par l'Université de Seoul appartient au domaine de l'hyperthermie magnétique. Ce document présente un mode d'activation sélective et de chauffage des particules magnétiques comprenant :
a) l'application d'un premier champ magnétique non-oscillant ou DC pour définir la fréquence de résonance des particules,
b) l'application d'un second champ magnétique haute fréquence appliqué à un angle donné par rapport au champ DC de façon à induire une précession de l'aimantation qui constitue l'activation des particules, qui, dans ce contexte correspond à un effet de chauffage. La fréquence du champ magnétique entraînant l'activation est de l'ordre de 1 MHz. Les particules magnétiques ont une structure en vortex, pouvant être constituées de maghémite (γ-Fe₂O₃), magnétite (Fe₃O₄), de ferrite de baryum (BaFeO) ou de CoFe₂O₄. Les particules ont un diamètre entre 40 nm et 200 nm.

La demande de brevet WO 2001017611, « Dispositif à but thérapeutique sur les tissus humains, destiné à influencer des particules magnétiques injectées au moyen d'un champ électromagnétique alternatif à gradient » appartient aussi au domaine de l'hyperthermie. Ce document décrit un dispositif générant un champ alternatif haute fréquence (jusqu'à 30 MHz) pour augmenter l'effet de chauffage et/ou cinétique des particules. Les particules utilisées sont constituées d'oxyde de fer et possèdent un diamètre compris entre 0.1 nm et 300 nm.

Il est également possible de distinguer les particules magnétiques connues dans l'état de l'art selon la technique utilisée pour les fabriquer. On distingue alors les techniques descendantes (ou « top-down approach» selon la terminologie anglaise) et ascendantes (bottom-up selon la terminologie anglaise).

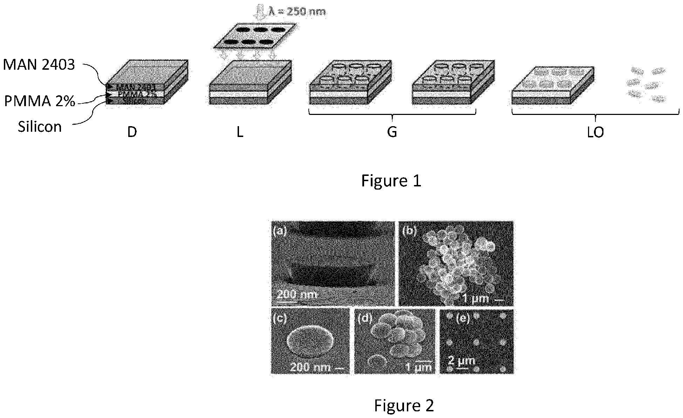

La figure 1 illustre schématiquement une méthode « top-down » (voir par exemple les documents « Self-polarization phenomenon and control of dispersion of synthetic antiferromagnetic nanoparticles for biological applications », publié dans Applied Physics Letters 97, 253112 (2010) de H. Joisten et al. et le document « Ferromagnetic microdisks as carriers for biomédical applications », publié dans Journal of Applied Physics 105, 07B306 (2009) de E. A. Rozhkova et al). Les principales étapes consistent à déposer des couches de résines D, à définir les particules par lithographie L, à développer et ouvrir la résine et déposer des films ou des multicouches magnétiques G, et à libérer les particules par lift-off LO. Plusieurs variantes sont possibles mais toutes procèdent de telles approches top-down. Les particules ainsi obtenues sont plates, souvent circulaires (sous forme de disques), avec une grande dimension de l'ordre du micron et d'épaisseur de l'ordre de 100 nm.

Du fait des techniques mises en oeuvre, la fabrication des particules est extrêmement coûteuse. En outre, cette technique présente un faible rendement de production par rapport aux besoins: de l'ordre de quelque 100 µg par substrat (« wafer ») sont obtenus, alors qu'il faudrait quelques grammes de particules pour traiter une tumeur de plusieurs cm³. Ce faible rendement est inhérent au processus de fabrication à la surface d'un substrat. La faible quantité produite est néanmoins suffisante pour les tests in vitro, permettant l'optimisation initiale des particules.

Du fait de la lithographie, ces particules ont une très faible dispersion en forme et en taille. Cette caractéristique est intéressante pour des applications de type agents de contraste pour l'imagerie magnétique mais n'est pas requise pour exercer des forces ou des couples sur des espèces biologiques, en particulier des cellules, ce qui contribue au fait que ce mode d'élaboration est inadapté par rapport aux besoins.

Une contrainte forte pour ces particules est la nécessité d'assurer leur dispersion dans un liquide. En pratique, une condition nécessaire est que l'aimantation en l'absence de champ appliqué soit faible. Pour des particules microniques, ceci est obtenu lorsque la structure magnétique est telle que l'aimantation interne, à champ nul, est compensée sur l'ensemble de la particule. Ceci peut être réalisé de plusieurs façons : soit en utilisant des particules présentant une configuration micromagnétique dite à fermeture de flux, soit en utilisant des structures en domaines désordonnés conduisant à une aimantation nulle comme par exemple dans les disques de magnétite polycristallins.

Concernant les particules à fermeture de flux, cette fermeture du flux magnétique peut être obtenue par exemple dans des disques d'alliage Ni80Fe20, de taille micronique et d'épaisseur de l'ordre de la centaine de nanomètres. Des exemples de particules à fermeture de flux sont illustrés à la figure 2. Dans ces structures, la configuration magnétique en champ nul est un vortex contenu dans le plan de la particule, avec pour seule aimantation rémanente la faible aimantation du coeur de vortex, voir par exemple le document « Biofunctionalized magnetic-vortex microdiscs for targeted cancer-cell destruction » publié dans Nature Materials 9, 165 (2010), de D. Kim et al.

En pratique, l'obtention de la structure vortex est limitée à une gamme réduite de tailles et de formes, qui exige pour la réalisation des particules d'utiliser les moyens d'élaboration top-down déjà évoqués. La susceptibilité de ces particules est suffisamment élevée pour que sous application d'un champ magnétique modéré (quelques dizaines de milliteslas), une aimantation nette apparaisse, pouvant engendrer un couple magnéto-mécanique significatif.

Le nickel contenu dans ces particules vortex n'étant pas un matériau biocompatible, les particules sont encapsulées entre deux films d'or lors de la fabrication. Les films d'or qui couvrent les deux faces des particules vortex permettent en outre la fonctionnalisation, notamment par l'emploi de thiols.

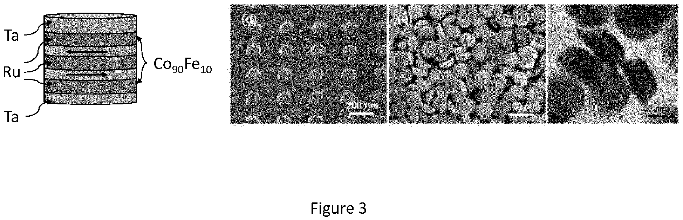

Un autre exemple de particules à fermeture de flux magnétique est constitué de multicouches dites antiferromagnétiques synthétiques, ou synthetic antiferromagnetic (SAF), dans lesquelles alternent un matériau magnétique et un matériau métallique non magnétique assurant un couplage antiferromagnétique entre les couches magnétiques adjacentes. Un exemple est constitué par les multicouches CoFe/Ru. Des exemples de ces particules sont illustrés à la figure 3 (voir aussi les documents « Self-polarization phenomenon and control of dispersion of synthetic antiferromagnetic nanoparticles for biological applications », publié dans Applied Physics Letters 97, 253112 (2010) de H. Joisten et le document "High-Moment Antiferromagnetic Nanoparticles with Tunable Magnetic Properties", publié dans Advanced Materials 20, 1479 (2008) de W. Hu et al).

L'épaisseur du métal non magnétique (le plus souvent du ruthénium) est choisie de telle sorte qu'un couplage antiferromagnétique se développe entre les couches magnétiques. Cette épaisseur est pour cela typiquement choisie entre 0.3 et 0.9 nm. En absence de champ, les aimantations des couches magnétiques successives sont antiparallèles et l'aimantation globale est nulle - on parle alors d'antiferromagnétique synthétique, SAF. Ces particules ont l'avantage de présenter une susceptibilité supérieure aux particules vortex d'où, pour un champ magnétique donné, un plus grand couple magnétique exercé (voir le document « Comparison of dispersion and actuation properties of vortex and synthetic antiferromagnetic particles for biotechnological applications », publié dans Applied Physics Letters 103, 132412 (2013) de S. Leulmi et al).

Ici encore, le ruthénium et le cobalt ou le nickel contenu dans ces particules SAF n'étant pas biocompatibles, les particules sont encapsulées entre deux films d'or lors de la fabrication.

Pour des disques circulaires (vortex ou SAF) libérés de leur substrat (c'est-à-dire par exemple en suspension dans un liquide), la minimisation de l'énergie dans un champ tournant conduit à orienter graduellement le plan de la particule parallèlement au plan de rotation du champ. La rotation de l'aimantation dans le plan de la particule circulaire n'exerce plus (dans ce cas) qu'un très faible couple magnétique.

Pour circonvenir à cet effet il est possible d'utiliser des particules magnétiques globalement plates mais de forme non circulaire (par exemple elliptique) voire irrégulière, ou des particules présentant une anisotropie perpendiculaire au plan des couches de sorte que l'aimantation de la particule pointe spontanément hors du plan de la particule et non dans le plan. De telles particules à aimantation perpendiculaire et antiferromagnétiques synthétiques sont par exemple constituées de multicouches composées de Ta, Pt, CoFeB et Ru (voir par exemple le document "Highly tunable perpendicularly magnetized synthetic antiferromagnets for biotechnology applications", publié dans Applied Physics Letters 107, 012403 (2015), de T. Vemulkar et al).

Les particules à aimantation perpendiculaire sont fabriquées à l'aide des techniques top-down décrites précédemment. Par contre elles présentent un nombre de couches beaucoup plus important qui allongent significativement les temps de fabrication et sont extrêmement sensibles aux problèmes de rugosité des couches. Leurs coûts de fabrication sont encore supérieurs à celui des particules en vortex. Les particules à aimantation perpendiculaire sont elles aussi constituées de matériaux qui, pour certains, ne sont pas biocompatibles.

Aucune des particules de l'état de la technique ne comporte toutes les caractéristiques précédemment citées pour la destruction efficace de cellules cancéreuses par mise en vibration basse fréquence.

Même si les particules en vortex ont donné des bons résultats en termes de destruction de cellules cancéreuses, elles contiennent des matériaux non biocompatibles, ce qui rend leur utilisation très difficile voire impossible.

De façon générale, les particules magnétiques de l'état de la technique sont obtenues par des procédés d'élaboration en salle blanche, qui sont intrinsèquement lents, complexes et onéreux.

De nombreux types de particules magnétiques sont d'autre part fabriquées par synthèse chimique. Par contre, les particules ainsi réalisées sont très petites (jusqu'à 20 nm de diamètre), elles sont le plus généralement sphériques (parfois cubiques) et présentent des structures soit homogènes, soit concentriques, qui ne permettent pas d'obtenir les propriétés magnétiques désirées.

Actuellement, on ne dispose pas de particules magnétiques qui soient à la fois biocompatibles, magnétiquement anisotropes, de taille micronique, aptes à exercer des couples importants sur des espèces biologiques et pouvant être fabriquées en quantité importante à faible coût.

### RESUME GENERAL DE L'INVENTION

Pour résoudre au moins partiellement les problèmes de l'état de la technique, un premier objet de la présente invention est un procédé de fabrication d'un fluide biocompatible comprenant des particules d'oxydes métalliques magnétiques obtenues à partir de poudres. Les particules d'oxydes métalliques sont biocompatibles et peuvent être produites en grande quantité et à faible coût.

Un premier objet de l'invention est donc un procédé de fabrication d'un fluide biocompatible comportant une poudre de particules magnétiques de forme allongée possédant une anisotropie magnétique de forme et ayant une granulométrie finale, ladite granulométrie finale étant définie par une première taille moyenne des particules dans une première direction et une deuxième taille moyenne dans une deuxième direction différente de la première direction, la deuxième taille moyenne étant inférieure à 1,5 fois la première taille moyenne, ladite granulométrie finale étant en outre définie par une première largeur de distribution des premières tailles et une deuxième largeur de distribution des deuxièmes tailles, ledit procédé comprenant les étapes suivantes :
- A partir d'une poudre de particules magnétiques ayant une granulométrie initiale différente de la granulométrie finale, modification de la granulométrie initiale par broyage et/ou par frittage de la poudre jusqu'à obtention de la granulométrie finale ;
- Introduction de la poudre de particules magnétiques dans un fluide biocompatible ;
la première taille moyenne des particules magnétiques étant comprise entre 0.2 µm et 10 µm et la largeur de distribution des premières tailles représentant au moins 30% de la valeur de la première taille moyenne.

Selon un mode de réalisation la première taille moyenne des particules est avantageusement comprise entre 0.2 µm et 5 µm.

On entend par granulométrie d'une poudre la distribution statistique des tailles des particules formant la poudre. Par exemple, la granulométrie d'une poudre peut être caractérisée par la taille moyenne de ses particules. Un autre paramètre décrivant la granulométrie d'une poudre est la largeur de la distribution des tailles des particules autour de la taille moyenne.

On entend par particules magnétiques des grains d'oxydes métalliques, notamment d'oxydes de fer ferrimagnétiques. Des exemples de tels matériaux sont la magnétite Fe₃O₄ ou la maghémite γ-Fe₂O₃ ou un mélange de ces composés.

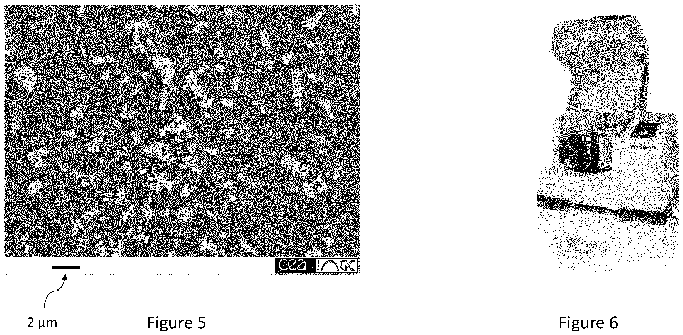

Les distributions de taille de particules peuvent être caractérisées par exemple par microscopie électronique à balayage comme illustré sur la Figure 5. Cette figure montre une assemblée de grains de magnétite de forme irrégulière.

On entend par taille dans une première direction la taille selon la plus grande dimension de chacune des particules. La taille moyenne dans une première direction est alors la moyenne de ces tailles selon la plus grande dimension de chaque particule. On entend ensuite par taille moyenne dans une deuxième direction différente de la première, la taille moyenne des particules dans une direction arbitraire transverse à la première direction de chaque particule. Ces tailles peuvent être obtenues par analyse d'images par microscopie électronique à balayage, comme illustré à la figure 5. Cette figure montre une assemblée de grains de magnétite de forme irrégulière. Une technique alternative consiste à déterminer les tailles par diffusion dynamique de la lumière (en anglais "Dynamic Light Scattering" - DLS").

On entend par fluide biocompatible un fluide qui n'interfère pas ni ne dégrade le milieu biologique dans lequel il est utilisé.

Avantageusement, ces matériaux sont biocompatibles et permettent des applications in-vivo du fluide biocompatible selon l'invention.

Avantageusement, ces particules ont une faible aimantation rémanente qui en assure la dispersion en phase liquide.

Avantageusement, les particules possèdent une anisotropie magnétique de forme qui vient de leur rapport d'aspect élevé. Cela permet une mise en vibration efficace par couple magnéto-mécanique en présence d'un champ magnétique variable.

L'étape d'obtention de la granulométrie finale à partir d'une granulométrie initiale est réalisée par broyage ou par frittage de la poudre possédant la granulométrie initiale.

Si la granulométrie initiale comporte une taille moyenne des particules supérieure à la première taille moyenne de la granulométrie finale, l'opération de broyage permet de réduire la taille moyenne des particules jusqu'à obtention de la granulométrie visée. L'opération de broyage peut par exemple être réalisée à l'aide d'un broyeur planétaire à bille.

Avantageusement, dans le cas d'un broyeur planétaire à billes de laboratoire, il est possible de fabriquer en une seule opération qui ne dure que quelques heures, des quantités de poudre qui vont de moins d'un gramme à plusieurs centaines de gramme.

Ces quantités sont de plusieurs ordres de grandeurs supérieures à celles obtenues par les approches top-down et couvrent largement les besoins pour le traitement en parallèle de plusieurs tumeurs ou tissus, chez l'animal ou chez l'homme.

Si la granulométrie initiale comporte une taille moyenne inférieure à la première taille moyenne de la granulométrie finale, la taille moyenne initiale des particules peut être augmentée parfrittage. On entend parfrittage le processus de fusion partielle ou totale des particules dû à l'échauffement de la poudre ou à l'application d'une pression élevée. L'opération de frittage s'effectue à des températures inférieures à la température de fusion du matériau composant les particules magnétiques.

Avantageusement il est possible d'appliquer à la poudre de départ possédant la granulométrie initiale une séquence d'opérations de broyage et de frittage afin d'obtenir la granulométrie finale. En d'autres termes, la taille moyenne des particules de la poudre broyée peut être augmentée par frittage si elle est trop petite. Vice-versa, la taille moyenne des particules de la poudre frittée peut être diminuée par broyage si elle est trop élevée.

Avantageusement, le procédé selon l'invention permet de produire des quantités de particules telles qu'on peut facilement mener en parallèle des études de caractérisation physico-chimique comme des mesures de taille et de potentiel de surface par DLS - Dynamic Light Scattering).

Le procédé selon l'invention peut s'inscrire dans un processus plus global dans lequel la poudre initiale serait issue d'une entité plus massive telle qu'un caillou ou un ruban.

La forme allongée des particules et leur anisotropie magnétique de forme sont telles que, sous application d'un champ magnétique uniforme et variable, elles subissent un couple magnétique qui induit une force appliquée sur le milieu où se trouve la particule ou sur le substrat sur lequel elle repose ou est fixée.

Avantageusement, il est possible d'utiliser la force appliquée par les particules magnétiques sur le milieu pour détruire des parties du milieu, telles que des cellules cancéreuses.

Grâce au fait que la largeur de distribution des premières tailles est supérieure ou égale au 30% de la valeur de la première taille moyenne, les particules ne sont pas de formes et de tailles homogènes. Cela permet, lors de la mise en vibration avec un champ magnétique d'amplitude et de fréquence données, de générer toute une gamme de couples et de forces mécaniques selon la taille de chacune des particules.

Avantageusement, ces particules sont bien adaptées à l'application de forces ou couples sur des espèces biologiques, surtout lorsque sont requis plusieurs milligrammes, voire plusieurs grammes de particules.

L'étape d'introduction de la poudre de particules magnétiques dans un fluide biocompatible se fait par transfert des particules magnétiques du bol de broyage ou frittage vers un fluide biocompatible. Comme exemple de fluides biocompatibles, on peut citer le sérum physiologique ou le PDMS. Pour l'injection chez l'animal ou chez l'homme, ce fluide est par exemple une solution de PEG. Pour les études in vitro, les particules sont ajoutées directement au fluide de culture cellulaire. Un exemple de tel fluide est une solution de DMEM (Dulbecco Modified Eagle Médium) avec GlutaMax et 10% de sérum de veau foetal (SVF).

L'idée de l'invention résulte de l'observation que la magnétite et certains autres oxydes ou composés de fer présentent naturellement des caractéristiques magnétiques similaires à celles recherchées dans les particules vortex pour les applications biomédicales, dans une gamme de tailles et de formes bien plus étendues que celles connues dans l'état de la technique. Le procédé de fabrication selon l'invention exploite avantageusement le fait qu'une poudre de composés de fer, pour peu que la taille moyenne des particules soit compatible avec l'application, est adaptée pour le déclenchement de la mort de cellules cancéreuses par vibration mécanique, sans qu'un contrôle strict de l'homogénéité de forme ni qu'une taille précisément définie soient nécessaire. Comparées aux particules vortex fabriquées par méthode top-down, les poudres utilisées dans le procédé selon l'invention sont beaucoup plus faciles et économiques à produire et leur mise en oeuvre est facilitée par leur biocompatibilité.

Le procédé selon l'invention peut également présenter une ou plusieurs des caractéristiques ci-dessous, considérées individuellement ou selon toutes les combinaisons techniquement possibles :
- la première taille moyenne est comprise entre 0.2 µm et 5 µm ;
- lors de l'étape de modification de la granulométrie initiale, le broyage de la poudre de particules magnétiques ayant la granulométrie initiale est suivi par le frittage de la poudre résultante du broyage ou le frittage de la poudre de particules magnétiques ayant la granulométrie initiale est suivi par le broyage de la poudre résultante du frittage ;
- la poudre de granulométrie finale est de même nature chimique que la poudre de granulométrie initiale ;
- le procédé selon l'invention comprend une étape de fonctionnalisation chimique des particules ;
- l'étape de fonctionnalisation chimique comporte une encapsulation d'au moins une partie des particules dans une couche inorganique ;
- la couche inorganique est en silice ;
- l'étape de fonctionnalisation chimique comprend une étape de greffage de polymères sur la surface des particules ou de la couche inorganique ;
- le polymère greffé comprend du polyéthylène glycol (PEG) ;
- les particules magnétiques sont des grains comprenant un oxyde de métal ;
- le procédé selon l'invention comprend en outre une étape consistant à affiner la distribution de taille des particules en solution.

Un autre objet de l'invention est un fluide biocompatible comportant une poudre de particules magnétiques de forme allongée possédant une anisotropie magnétique de forme et ayant une granulométrie finale, ladite granulométrie finale étant définie par une première taille moyenne des particules dans une première direction et une deuxième taille moyenne dans une deuxième direction différente de la première direction, la deuxième taille moyenne étant inférieure à 1,5 fois la première taille moyenne, ladite granulométrie finale étant en outre définie par une première largeur de distribution des premières tailles et une deuxième largeur de distribution des deuxièmes tailles, la première taille moyenne des particules magnétiques étant comprise entre 0.2 µm et 10 µm et la largeur de distribution des premières tailles représentant au moins 30% de la valeur de la première taille moyenne.

Selon un mode de réalisation, la première taille moyenne des particules est avantageusement comprise entre 0.2 µm et 5 µm.

La grandeur du couple magnéto-mécanique dépend de l'anisotropie magnétique. Les particules doivent posséder une anisotropie magnétique élevée. Comme cette anisotropie a souvent pour origine l'anisotropie de forme de la particule, une particule sphérique ne serait pas adaptée pour les applications visées.

Si la particule est attachée à une espèce biologique, le couple qu'elle subit induit, par effet levier, une force mécanique. Le couple sur une particule est proportionnel au moment magnétique, donc à son volume : tout considéré une particule plus grosse est préférable. La force mécanique résultant du couple dépend aussi d'autres facteurs : 1) d'une part du bras de levier selon lequel le couple s'exerce, donc de la forme de particule ; 2) dans le cas où la particule est ancrée à l'espèce biologique, la force mécanique transmise dépend aussi de la force de liaison. Cet ancrage peut être obtenu par la fonctionnalisation de la particule avec un ligand adéquat.

Les particules qui permettent d'exercer ces actions mécaniques doivent être suffisamment grosses pour perturber les espèces biologiques visées. Les particules magnétiques utilisées dans le fluide biocompatible selon l'invention sont de l'ordre du micron et permettent d'agir sur des cellules dont la taille est de l'ordre de la dizaine de microns.

Les particules magnétiques utilisées dans le fluide biocompatible selon l'invention, possèdent plusieurs avantages :
- Grâce à leur faible aimantation rémanente les particules ne s'agglomèrent pas de façon irréversible lorsqu'elles sont en solution, voir par exemple « Self-polarization phenomenon and control of dispersion of synthetic antiferromagnetic nanoparticles for biological applications », publié dans Applied Physics Letters 97, 253112 (2010) de H. Joisten et al. ;
- Les particules sont constituées par des matériaux magnétiques biocompatibles pour ne pas induire de réaction toxique autre que celle induite par les effets magnéto-mécaniques recherchés dans l'organisme visé ;
- Las particules peuvent être fonctionnalisées, par exemple pour en piloter la mobilité, pour assurer la stabilité dans le milieu où elles sont insérées, pour en prévenir l'agglomération et/ou permettre une action ciblée, notamment thérapeutique.

D'autres part, la forme exacte de la particule, de même que la régularité de tailles et de formes lors d'une réalisation de l'invention ne sont pas des paramètres nécessitant un contrôle fin. Le couple magnéto-mécanique et les forces résultantes dépendent plus généralement du rapport d'aspect et des dimensions globales. Dans la plupart des particules magnétiques connues dans l'état de l'art, l'optimisation des propriétés magnétiques nécessite un contrôle rigoureux de la taille et de la forme des particules. Par exemple dans le cadre d'agents de contraste pour l'imagerie par résonance magnétique, on veut que les inhomogénéités observées dans les temps de relaxation de spin des protons soient causées par les variations de densité des tissus environnants et le moins possible par les distributions de propriétés des agents de contraste eux-mêmes. Avantageusement, la présente invention relaxe cette contrainte et admet l'utilisation d'un ensemble de particules de forme et de tailles dispersées, sans préjudice pour la réalisation de l'objectif thérapeutique recherché.

Le fluide biocompatible selon l'invention peut également présenter une ou plusieurs des caractéristiques ci-dessous, considérées individuellement ou selon toutes les combinaisons techniquement possibles :
- la première taille moyenne des particules est comprise entre 0.2 µm et 5 µm ;
- les particules magnétiques sont des grains comprenant un oxyde de métal ;
- l'oxyde de métal est un oxyde de fer ferrimagnétique choisi dans un groupe comprenant : magnétite, maghémite ou une combinaison de ces matériaux ;
- les particules magnétiques sont fonctionnalisées chimiquement ;
- la fonctionnalisation chimique comporte l'encapsulation d'au moins une partie des particules magnétiques dans une couche inorganique ;
- la couche inorganique est en silice ;
- la fonctionnalisation chimique comprend une étape de greffage de polymères sur la surface des particules ou de la couche inorganique ;
- le polymère greffé comprend du polyéthylène glycol (PEG) ;
- les particules sont des grains comprenant un oxyde de métal.

### LISTE DES FIGURES

D'autres caractéristiques de l'invention ressortiront clairement de la description qui en est donnée ci-dessous, à titre indicatif et nullement limitatif, en référence aux figures, parmi lesquelles :
- La figure 1 représente schématiquement l'approche de fabrication de particules magnétiques de type « top down » selon l'état de la technique ;
- La figure 2 représente des images par microscopie électronique à balayage de particules magnétiques réalisées à l'aide de la technique illustrée à la figure 1 ;
- La figure 3 illustre des exemples de particules magnétiques de type SAF selon l'état de la technique ;
- La figure 4 illustre schématiquement les étapes du procédé de fabrication d'un fluide biocompatible selon l'invention ;
- La figure 5 est une image au microscope électronique des particules du fluide biocompatible selon l'invention ;
- La figure 6 représente un broyeur à billes utilisé pour modifier la granulométrie d'une poudre ;
- La figure 7 illustre schématiquement la fonctionnalisation des particules de magnétite avec des organosilanes ;
- La figure 8 illustre schématiquement la fonctionnalisation de particules magnétiques avec des fluorophores biotine/streptavidine-PE ;
- La figure 9 montre à gauche des particules selon l'invention fonctionnalisées avec un fluorophore et à droite des particules selon l'invention non-fonctionnalisées ;
- La figure 10 illustre le champ magnétique uniforme au centre d'un cylindre de Hallbach.

### DESCRIPTION DETAILLEE

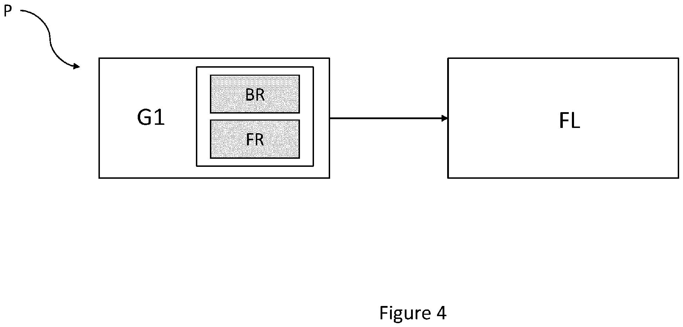

La figure 4 illustre schématiquement les étapes du procédé de fabrication d'un fluide biocompatible comprenant des particules magnétiques selon l'invention.

Lors de l'étape G1, une poudre de particules ayant une granulométrie finale et destinée à être dispersée dans un fluide biocompatible est obtenue à partir d'une poudre initiale de particules magnétiques.

La granulométrie finale est caractérisée par une première taille moyenne des particules dans une première direction et une deuxième taille moyenne des particules dans une deuxième direction.

La poudre initiale possède une granulométrie initiale caractérisée par une taille moyenne des particules. Les particules de la poudre initiale peuvent avoir elles aussi une forme allongée.

Si la taille moyenne des particules de la poudre initiale est supérieure aux tailles moyennes caractéristiques de la granulométrie finale ciblée, la poudre finale est obtenue par broyage BR de la poudre initiale.

Par exemple, il est possible de broyer une poudre initiale de particules de magnétite ayant une taille moyenne initiale de 5 µm pour obtenir une poudre finale de particules de magnétite ayant une taille moyenne de 2 µm.

Alternativement, si la poudre initiale possède une taille moyenne des particules plus petite que les taille moyennes de la granulométrie cible, il est possible d'obtenir la poudre finale par frittage FR de la poudre initiale.

Avantageusement, les étapes de broyage BR et de frittage FR peuvent être exécutées en séquence, pour ajuster la granulométrie de la poudre jusqu'à obtention de la granulométrie finale désirée.

L'anisotropie des formes obtenues résulte à la fois de la dispersion en taille et en forme de la poudre initiale et du caractère aléatoire des chocs engendrant la fracturation des grains. Dans le cas où les particules initiales ont un rapport de forme allongé, les particules obtenues après un broyage modéré conservent un rapport de forme allongé (même si ce rapport de forme peut diminuer).

Par exemple, si la poudre obtenue après l'étape de broyage comporte une taille moyenne des particules trop petite, il est possible d'augmenter la taille des particules par frittage.

Une fois la poudre finale obtenue, elle est transférée dans un fluide biocompatible lors de l'étape FL.

Le transfert FL peut être effectué par capture des particules contre la paroi d'un contenant à l'aide d'un aimant ou d'un champ magnétique, vidange du liquide initial et ajout d'un fluide biocompatible.

Une variante consiste à récupérer les particules en plongeant dans le contenant où elles se trouvent un dispositif magnétique dont le champ rayonné peut être activé ou désactivé. Les particules seront attirées et maintenues contre les parois du dispositif lorsque le champ rayonné est activé et relarguées dans un second liquide biocompatible lorsque le champ rayonné est désactivé.

Selon un mode de réalisation, le procédé P comprend en outre une étape de modification de la granulométrie des particules en solution, après broyage ou frittage et le transfert de la poudre dans le fluide biocompatible. Cette étape consiste à affiner la distribution de taille des particules en solution notamment par filtration à l'aide de filtres-seringues ou de filtres papier, par suspension/décantation, par séparation magnétique, ou par centrifugation.

La figure 5 représente une image au microscope électronique des particules magnétiques utilisées dans le procédé P selon l'invention. La poudre présente une granulométrie finale obtenue à partir d'une granulométrie initiale par broyage/frittage. Les particules comprises dans la poudre finale possèdent des formes irrégulières.

A l'appui de l'invention, on observe que les mesures d'aimantation sur des cristaux de magnétite synthétique, avec des tailles entre 0.3 µm et 30 µm, ou sur des échantillons massifs de magnétite d'origine naturelle, ou sur des plots lithographiés jusqu'à des diamètres de 300 nm indiquent une rémanence de l'aimantation inférieure à 0.2. Voir par exemple, les documents « Grain size dependence of low-temperature remanent magnetization in natural and synthetic magnetite : Experimental study » publié dans Earth Planet Space 61, 119 (2009) de A. V. Smirnov et al.

Ces différents éléments indiquent que les propriétés magnétiques de la magnétite sont adaptées à l'utilisation visée, que ces propriétés sont robustes au regard de la forme et de la taille, et peu sensibles aux conditions particulières d'élaboration.

La figure 6 représente un broyeur à bille, connu de l'homme du métier. Un tel broyeur à billes est utilisé pour modifier la granulométrie initiale en réduisant la taille des particules.

Les broyeurs planétaires à billes permettent, dans le cas des modèles de laboratoire, de fabriquer en une seule opération, qui ne dure que quelques heures, des quantités de poudre qui vont de moins d'un gramme (avec un seul bol de 12 ml) à plusieurs centaines de grammes (lorsque plusieurs bols de 500 ml sont utilisés en parallèle). Ces quantités sont de plusieurs ordres de grandeurs supérieures à celles obtenues par les approches top-down et couvrent largement les besoins pour le traitement en parallèle de plusieurs tumeurs ou tissus, chez l'animal ou chez l'homme. D'autre part les quantités sont telles qu'on peut facilement mener en parallèle des études de caractérisation physico-chimique (p. ex. mesures de taille et de potentiel de surface par DLS - Dynamic Light Scattering), ou des études de fonctionnalisation chimique requérant plusieurs milligrammes ou grammes de matière.

D'autres technologies, à l'exemple des broyeurs utilisés dans l'industrie pharmaceutique, permettent de broyer des charges de poudre de plusieurs kilos jusqu'à des granulométries microniques. De tels volumes relèvent d'une exploitation industrielle des particules, inenvisageable avec les approches top-down tant le coût de revient au gramme est élevé.

La composition de la poudre initiale est semblable à la composition des particules désirées. Une variante consiste à réaliser l'oxydation totale ou partielle d'une poudre de fer, ou à réaliser par broyage la mécanosynthèse d'un oxyde de fer.

Un exemple d'utilisation d'un broyeur à bille standard pour réduire modifier la granulométrie d'une poudre de magnétite est le suivant.

La poudre de magnétite est introduite dans un bol de broyage de 50 ml, en oxyde de zirconium, avec un certain nombre de billes de même matière, de diamètre centimétrique. Une caractéristique du bol et des billes est d'être constitués d'un matériau de dureté supérieure à celle de la poudre broyée. Par exemple, la dureté de l'oxyde de zirconium sur l'échelle de Mohs est de 8, celle de la magnétite est de 5.5.

Une seconde caractéristique du bol et des billes est de ne pas relarguer de contaminant toxique lors du broyage. Ceci est le cas de l'oxyde de zirconium, mais n'est pas le cas de l'acier (qui au broyage relargue du chrome). Une variante consiste à utiliser un bol et des billes d'une dureté autre, mais ne relarguant pas de contaminant toxique.

La charge en magnétite représente environ un tiers du volume du bol de broyage, ce qui représente en moyenne 2 g de magnétite pour un bol de 50 ml. Une certaine quantité de liquide peut être ajoutée à la charge pour en faciliter le broyage, par exemple 20 ml d'isopropanol. D'autres adjuvants peuvent être ajoutés, en quantité variable, par exemple de l'acide oléique ou stéarique qui favorisent la dispersion des particules broyées.

Une variante consiste à ajouter une certaine quantité d'eau pour induire une réaction d'oxydation des particules lors du broyage et en modifier la composition chimique.

Le bol est fermé hermétiquement par un couvercle. Le broyage résulte de la mise en rotation excentrée du bol, par exemple à 600 rpm pendant 2 heures. Les temps de broyage et/ou l'énergie des billes sont ajustés en fonction des granulométries initiale et finale.

Une variante consiste à utiliser une autre technique ou appareil de broyage, différent par le type de mouvement imposé au bol de broyage et par la nature de l'impact des billes avec la poudre.

À la fin du broyage et en fonction des conditions utilisées, la granulométrie de la poudre est réduite, avec par exemple une distribution de taille telle que la plus grande dimension soit centrée sur 2 µm avec une distribution de 30% ou plus. La forme des particules est d'autre part irrégulière.

Selon un mode de réalisation de l'invention, le procédé P de fabrication d'un fluide biocompatible comprenant des particules magnétiques comporte en outre une étape de fonctionnalisation chimique des particules.

Très souvent, les particules sont fonctionnalisées à l'aide de composés qui procurent une stabilisation de la structure, une protection contre l'oxydation (notamment pour les particules de fer), une barrière stérique ou électrostatique à l'agglomération et/ou qui permettent de circuler dans un organisme ou un tissu, ou d'interagir avec un tissu biologique ou une cellule soit pour y adhérer, y pénétrer, ou y délivrer de façon ciblée un médicament ou toute autre substance active.

Avantageusement, le greffage de polymères ou l'encapsulation dans la silice, utilisés pour la stabilisation des particules, procurent une répulsion stérique. Si, de plus, la couche fonctionnalisée ou les chaînes polymères sont chargées, elles induisent une répulsion électrostatique entre particules, ce qui réduit les effets d'attraction magnétique et augmente l'effet de dispersion.

Par exemple, l'effet de répulsion stérique est obtenu par greffage de polyéthylène glycol (PEG), qui forme une couche d'épaisseur variable à la surface de la particule de 8 nm (PEG 1k) à 15 nm (PEG 5k). L'épaisseur de la couche de PEG greffée sur la particule impose une distance d'approche minimale entre les particules magnétiques.

Si on assimile chaque particule à un dipôle magnétique, l'énergie d'interaction magnétique diminue comme l'inverse du cube de la distance d entre les particules. Le greffage de molécules longues à la surface des particules diminue cet effet d'interaction. L'énergie d'interaction magnétique diminuant en 1/d³, cela en diminue l'effet.

Le greffage de PEG prévient aussi l'opsonisation des particules, ce qui réduit leur élimination par les phagocytes et prolonge leur durée de vie dans l'organisme, voir par exemple le document « Effect of polyethyleneglycol (PEG) chain length on the bio-nano-interactions between PEGylated lipid nanoparticles and biological fluids: from nanostructure to uptake in cancer cells », publié dans Nanoscale 6, 2782 (2014) de Pozzi et al.

Avantageusement, la fonctionnalisation de la particule peut permettre le transport de substances à visée thérapeutique, de même que le ciblage sélectif d'un tissu ou d'une cellule, par greffage d'anticorps. Cette fonctionnalisation est assurée par le greffage préalable de thiols (pour des particules recouvertes d'or - la plupart des particules actuelles) ou de groupes amine (pour les particules de magnétite de l'invention), voir par exemple le document « Functionalization of Fe3O4 NPs by Silanization », publié dans Materials. 9, 826 (2016) de S. Villa et al.

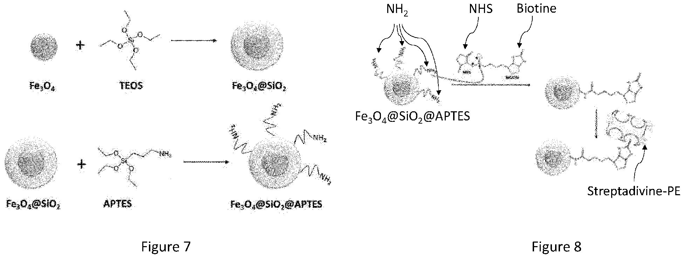

Selon un mode de réalisation particulière, la fonctionnalisation peut être obtenue par encapsulation dans une matrice inorganique de silice puis greffage d'un organosilane comme il est montré à la figure 7.

Le précurseur de silice est le tétraéthoxysilane (TEOS) et l'organosilane est le 3-aminopropyltriethoxysilane (APTES). Le groupement fonctionnel amine de l'APTES permet de maintenir le caractère hydrophile de la surface et de greffer une biomolécule.

L'encapsulation des particules avec la silice peut être réalisée de la façon suivante : dans un ballon Bicol, 6 mg de particules de Fe3O4, 20 ml d'éthanol absolu et 100 ml d'eau ultra pure, ultrasons 15 min à 40°C. Ajout successif de 400 µL d'eau ultra pure, 900 µL d'ammoniaque (28% aq.) et 120 µL de TEOS. Agitation à 40°C pendant 2 h.

La fonctionnalisation avec l'APTES est alors réalisée de la façon suivante : dans un ballon Bicol, 1 ml de Fe3O4@SiO2 (60 mg/L) dans de l'eau ultra pure ajoutés à 1 ml d'éthanol et à 43 µL d'APTES (2% v/v). Agitation à 50°C pendant 24 h.

L'efficacité de la fonctionnalisation des particules est vérifiée par le greffage d'un fluorophore, de la phycoérythrine (PE) couplée à la streptavidine. Le crosslinker Nhydroxysuccinimide-Biotine (NHS-Biotine) est utilisé pour la formation d'une liaison amide avec le groupement amine du APTES, puis la streptavidine-PE est liée à la biotine.

Pour une fonctionnalisation pour de la fluorescence, le procédé suivant peut être utilisé : dans un tube Eppendorf les particules sont laissées en contact avec 4 µL de NHS-Biotine (10 mM) et 396 µL de Tampon Phosphate Salin à pH 8 (PBS 8), 1h sous vortex. Rinçage trois fois avec PBS 8, deux fois avec PBS 7,4 et suspension dans 100 µL de PBS 7,4 puis agitation sous vortex. Ajout de 5 µL de Streptavidine-PE et agitation 15 minutes sous vortex à l'abri de la lumière. Rinçage trois fois avec PBS 7,4 puis dépôt sur lamelle de microscope pour observation avec lumière entre 520 et 550 nm.

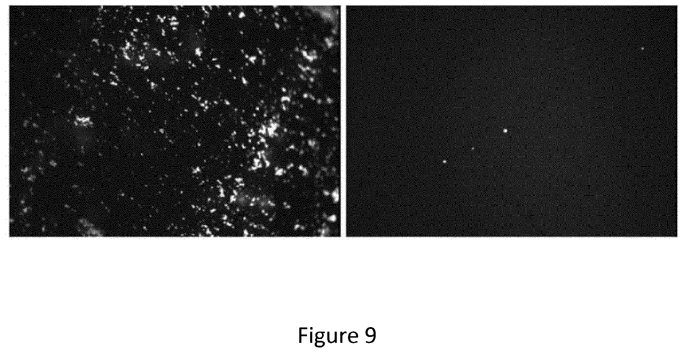

Les résultats de la fonctionnalisation pour de la fluorescence sont illustrés à la figure 9, qui montre :
- A gauche, image au microscope optique à fluorescence de la fonctionnalisation à l'APTES de particules de magnétite ;
- A droite, image au microscope optique à fluorescence de particules témoins non fonctionnalisées.

Sur ces figures une émission de fluorescence est observée uniquement pour les particules fonctionnalisées, ce qui confirme l'efficacité de la fonctionnalisation.

Les particules destinées à être mises en présence de tissus vivant ou de cellules sont, au sortir du broyeur où elles sont dispersées dans l'isopropanol, conditionnées de la façon suivante. Les particules sont attirées au fond d'un contenant où elles se trouvent au moyen d'un aimant ; la plus grande partie de l'isopropanol est retiré à l'aide d'une pipette : l'isopropanol est remplacé par de l'éthanol ; toujours en attirant les particules au fond du contenant et en pipetant le liquide, trois rinçages à l'aide du milieu de culture sont effectués.

Les particules sont mises en présence des cellules ou tissus par ajout direct, au récipient ils se trouvent, de la solution particule - milieu de culture décrit. Une période d'incubation, par exemple 24 heures, peut être respecté entre la mise en présence des particules et des tissus ou des cellules pour permettre la diffusion des particules dans le milieu et/ou le greffage ou l'incorporation des particules sur les espèces visées.

Les particules destinées à des observations microscopiques, ou à des mesures ou caractérisations où il est souhaitable qu'elles soient dispersées sur une surface (p. ex. les mesures magnétiques), sont, au sortir du broyeur où elles sont dispersées dans l'isopropanol, dispersées de la façon suivante. L'isopropanol est remplacé, par la technique précédemment décrite, par un solvant inerte à pression de vapeur élevée (p. ex. l'acétone). Le substrat destiné à recevoir les particules, par exemple un substrat de silicium de l'ordre du centimètre carré, est placé dans un champ magnétique aussi élevé que possible, perpendiculaire à sa surface. Ceci peut être réalisé en posant le substrat sur un aimant permanent puissant. Si possible, le substrat est chauffé à une température légèrement inférieure à la température d'ébullition du solvant. Dans le cas de l'acétone à pression ambiante, cette température peut être de 50°C ; Une goutte de particules en solution est déposée rapidement sur le substrat. Si le mouillage de la goutte est rapide et si l'évaporation de la goutte survient rapidement : 1) l'épaisseur de la goutte qui s'étale/s'évapore reste faible ; 2) la formation de chaînes de particules, dont l'orientation magnétique serait ici perpendiculaire à la surface, est limitée par la rapide dispersion à la surface du substrat et la faible épaisseur de liquide en évaporation. La vitesse d'étalement de la goutte peut aussi, selon la nature du solvant, être accélérée par un traitement de surface qui augmente l'affinité substrat/liquide.

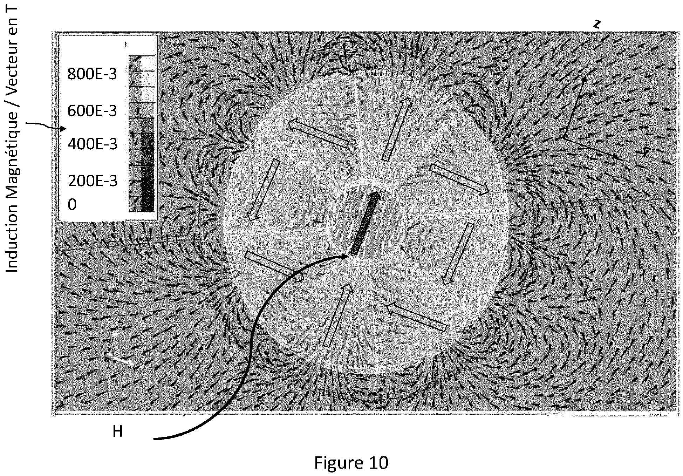

La mise en vibration des particules (préalablement conditionnées et mises en présence des tissus ou cellules à traiter selon la méthode décrite) est obtenue en les soumettant à un champ variable en module et/ou en direction. Une solution est d'utiliser un cylindre de Halbach illustré à la figure 10. Ce cylindre est composé d'aimants permanents disposés en secteurs et comporte en son centre une cavité cylindrique où règne un champ magnétique homogène H, de l'ordre de quelques dixièmes de teslas, orienté perpendiculairement à l'axe du cylindre. L'échantillon de tissus biologique ou de cellules à traiter, avec les particules magnétiques, est placé au centre du cylindre à l'aide du support approprié, selon qu'il s'agit de cellules de culture, de tissus vivants, ou éventuellement d'une souris.

La mise en rotation du cylindre génère un champ tournant qui fait osciller les particules placées dans la cavité, et engendrera un couple magnéto-mécanique.

Un autre objet de l'invention est un fluide biocompatible comportant une poudre de particules magnétiques de forme allongée. La forme allongée des particules détermine une anisotropie magnétique de forme ce qui permet leur mise en vibration grâce à l'application d'un champ magnétique variable dans le temps.

La poudre de particules magnétiques possède une granulométrie finale définie par une première taille moyenne des particules dans une première direction et une deuxième taille moyenne des particules dans une deuxième direction. La granulométrie finale est définie en outre par une première largeur de distribution des premières tailles et une deuxième largeur de distribution des deuxièmes tailles. La première taille moyenne des particules est comprise entre 0.2 µm et 5 µm. La largeur de distribution des premières tailles est supérieure ou égale à 30% de la valeur de la première taille moyenne.

Avantageusement, l'anisotropie magnétique de forme permet la mise en vibration efficace des particules en présence d'un champ magnétique variable dans le temps.

Selon un mode de réalisation, la deuxième taille moyenne est inférieure à 1,5 fois la première taille moyenne.

Avantageusement, cette différence entre la première taille moyenne et la deuxième taille moyenne permet d'obtenir une anisotropie magnétique de forme élevée et donc d'augmenter le couple magnéto-mécanique en présence d'un champ magnétique externe variable.

Selon un mode de réalisation, les particules magnétiques sont réalisées en oxyde de fer.

Selon un mode de réalisation, l'oxyde de fer ferrimagnétique est choisi dans un groupe comprenant : magnétite, maghémite ou une combinaison de ces matériaux. Avantageusement, ces matériaux sont biocompatibles et adaptés à des applications de type destruction de cellules ou tissus cancéreux humains ou animaux.

Les particules magnétiques présentes dans le fluide biocompatible peuvent en outre être fonctionnalisées chimiquement, comme expliqué en référence à l'étape de fonctionnalisation du procédé selon l'invention.

Le fluide biocompatible selon l'invention peut être utilisé pour la destruction de cellules cancéreuses par vibrations magnéto-mécaniques selon le processus expérimental suivant.

Les particules magnétiques sont transférées dans un liquide biocompatible, avec une concentration typique de 10⁷ particules/ml.

Les particules peuvent être fonctionnalisées.

La fonctionnalisation peut consister au greffage d'un composé permettant l'accroche ciblée de la particule sur un tissu, une cellule ou un site préférentiel de la paroi cellulaire.

Le composé greffé peut être un anticorps, qui permet à la particule de se fixer à la surface de certaines cellules spécifiques.

La fonctionnalisation peut consister au greffage d'un composé qui assure ou favorise l'endocytose des particules, par les cellules ciblées.

La fonctionnalisation peut avoir pour but d'assurer une meilleure circulation et une meilleure durée de vie de la particule dans l'organisme ou le tissu, ou au contraire avoir pour but d'en diminuer la mobilité et assurer le maintien de la particule au plus proche de l'endroit où elle a été positionnée, par exemple lors de l'injection au sein d'un tissu.

La localisation de la particule à l'endroit où doit s'exercer la vibration magnéto-mécanique est réalisée par un ou plusieurs des moyens suivants : fonctionnalisation ciblée ; déplacement des particules sous l'effet d'un gradient de champ magnétique, qu'il soit interne ou externe ; injection directe au sein du tissus à traiter.

Lorsque les particules sont en place, elles sont mises en vibration par l'application d'un champ externe variable. Un exemple de réalisation est l'utilisation d'un cylindre de Halbach en rotation. Le cylindre de Halbach génère dans une cavité cylindrique un champ magnétique, perpendiculaire à l'axe de la cavité. La mise en rotation du cylindre crée un champ tournant.

L'intensité du champ magnétique est de l'ordre de 0.2 T à 1 T. La fréquence de rotation du champ magnétique est de l'ordre de 10 Hz à 30 Hz. La durée d'un traitement est de l'ordre de 5 minutes à 1 heure.

Le traitement peut être réalisé sur ces cellules de cultures. Ces cellules sont par exemple issues de lignées de cellules cancéreuses, humaines ou animales. Les cellules sont mises en présence des particules, avant de réaliser le traitement, pour un temps d'incubation d'une durée typique de 24 heures.

Les cellules et les particules peuvent être placées dans des puits, dans un liquide nutritif adapté.

Les cellules et les particules peuvent être intégrées à un gel, ou à une structure leur procurant un substrat de croissance tridimensionnel.

Le but de l'application des vibrations magnéto-mécaniques est de déclencher la mort cellulaire sous application de vibrations magnéto-mécaniques à l'intérieur de la cellule, à la surface de la cellule, ou dans le milieu environnant la cellule. Les cellules particulièrement visées par cette application sont les cellules cancéreuses.

Une variante de l'application des vibrations magnéto-mécaniques peut être de modifier ou d'orienter la division cellulaire, ou de modifier ou d'orienter la croissance de tissus. Cette application vise à promouvoir la régénération de tissus par la stimulation de leur croissance, notamment ceux de la moelle épinière.

## Revendications

1. Procédé de fabrication d'un fluide biocompatible comportant une poudre de particules magnétiques de forme allongée possédant une anisotropie magnétique de forme et ayant une granulométrie finale, ladite granulométrie finale étant définie par une première taille moyenne des particules dans une première direction et une deuxième taille moyenne dans une deuxième direction différente de la première direction, la deuxième taille moyenne étant inférieure à 1,5 fois la première taille moyenne, ladite granulométrie finale étant en outre définie par une première largeur de distribution des premières tailles et une deuxième largeur de distribution des deuxièmes tailles, ledit procédé comprenant les étapes suivantes :
- A partir d'une poudre de particules magnétiques ayant une granulométrie initiale différente de la granulométrie finale, modification (G1) de la granulométrie initiale par broyage et/ou par frittage de la poudre jusqu'à obtention de la granulométrie finale ;
- Introduction (FL) de la poudre de particules magnétiques dans un fluide biocompatible ;
La première taille moyenne des particules magnétiques étant comprise entre 0.2 µm et 10 µm et la largeur de distribution des premières tailles représentant au moins 30% de la valeur de la première taille moyenne.

2. Procédé de fabrication d'un fluide biocompatible selon la revendication précédente **caractérisé en ce que** la première taille moyenne des particules est comprise entre 0.2 µm et 5 µm.

3. Procédé de fabrication d'un fluide biocompatible selon l'une des revendications précédentes **caractérisé en ce que** lors de l'étape de modification (G1) de la granulométrie initiale, le broyage de la poudre de particules magnétiques ayant la granulométrie initiale est suivi par le frittage de la poudre résultante du broyage ou le frittage de la poudre de particules magnétiques ayant la granulométrie initiale est suivi par le broyage de la poudre résultante du frittage.

4. Procédé de fabrication d'un fluide biocompatible selon l'une des revendications précédentes **caractérisé en ce que** la poudre de granulométrie finale est de même nature chimique que la poudre de granulométrie initiale.

5. Procédé de fabrication d'un fluide biocompatible selon l'une des revendications précédentes **caractérisé en outre en ce qu'**il comprend une étape de fonctionnalisation chimique des particules.

6. Procédé de fabrication d'un fluide biocompatible selon la revendication précédente **caractérisé en ce que** l'étape de fonctionnalisation chimique comporte une encapsulation d'au moins une partie des particules dans une couche inorganique.

7. Procédé de fabrication d'un fluide biocompatible selon la revendication précédente **caractérisé en ce que** la couche inorganique est en silice.

8. Procédé de fabrication d'un fluide biocompatible selon l'une des revendications 5 à 7 **caractérisé en ce que** l'étape de fonctionnalisation chimique comprend une étape de greffage de polymères sur la surface des particules ou de la couche inorganique.

9. Procédé de fabrication d'un fluide biocompatible selon la revendication précédente **caractérisé en ce que** le polymère greffé comprend du polyéthylène glycol (PEG).

10. Procédé de fabrication d'un fluide biocompatible selon l'une des revendications précédentes **caractérisé en ce que** les particules magnétiques sont des grains comprenant un oxyde de métal.

11. Procédé de fabrication d'un fluide biocompatible selon l'une des revendications précédentes **caractérisé en ce qu'**il comprend en outre une étape consistant à affiner la distribution de taille des particules en solution.

12. Fluide biocompatible comportant une poudre de particules magnétiques de forme allongée possédant une anisotropie magnétique de forme et ayant une granulométrie finale, ladite granulométrie finale étant définie par une première taille moyenne des particules dans une première direction et une deuxième taille moyenne dans une deuxième direction différente de la première direction, la deuxième taille moyenne étant inférieure à 1,5 fois la première taille moyenne, ladite granulométrie finale étant en outre définie par une première largeur de distribution des premières tailles et une deuxième largeur de distribution des deuxièmes tailles, la première taille moyenne des particules magnétiques étant comprise entre 0.2 µm et 10 µm et la largeur de distribution des premières tailles représentant au moins 30% de la valeur de la première taille moyenne.
